# EUROPÄISCHE PATENTANMELDUNG

(11) **EP 2 133 065 A2**
(43) Veröffentlichungstag der Anmeldung: **16.12.2009**
(21) Anmeldenummer: 09162243.1
(22) Anmeldetag: 09.06.2009
(51) Int. Cl.: A61K 8/02, A61K 8/04, A61K 8/06, A61K 8/19, A61K 8/24, A61K 8/34, A61K 8/60, A61K 8/67, A61K 8/73, A61K 8/97, A61Q 19/00

(54) **Kosmetisches Verfahren zur Hautbehandlung**

(30) Priorität: 09.06.2008 AT 9252008
(71) Anmelder: PATERNOSTER Leopoldine, 3270 Scheibbs (AT); Styx Naturcosmetic GmbH, 3200 Ober-Grafendorf (AT)
(72) Erfinder: Paternoster, Leopoldine, 3270, Scheibbs (AT); Stix, Wolfgang, 3200, Ober-Grafendorf (AT)
(74) Vertreter: Sonn & Partner Patentanwälte

(57) **Zusammenfassung**

Die vorliegende Erfindung betrifft ein kosmetisches Verfahren zur Hautbehandlung, insbesondere zur Hautglättung und Hautreinigung, umfassend die Schritte:
a) Aufbringen einer flüssigen kosmetischen Zusammensetzung auf die Haut,
b) Aufbringen einer Creme in Form einer Wasser-in-Öl-Emulsion, eines Gels oder einer Lotion auf die Haut,
c) Abdecken der Haut mit einer Wasser- und Wasserdampf-undurchlässigen Beschichtung, wobei die Haut nach Schritt c) und gegebenenfalls vor Schritt a) wärmebehandelt und die Wasser- und Wasserdampf-undurchlässige Beschichtung an der Haut anliegend aufgebracht wird.

## Beschreibung

Die Haut in ihrer Funktion als Barriere gegenüber der Umwelt und als größtes Organ des menschlichen Körpers ist Umwelteinflüssen (z.B. UV-Strahlung, schädigenden Dämpfen) ohne ausreichende Schutzvorkehrungen (z.B. Abdeckung der Haut, Verwendung von kosmetischen Zubereitungen) direkt ausgesetzt. Derartige Umwelteinflüsse und der natürliche Alterungsprozess eines Individuums führen dazu, dass die Haut einer kontinuierlichen Alterung ausgesetzt ist, die z.B. zu einer verstärkten Faltenbildung führt. Der seit jeher bestehende Wunsch der Menschen, die biologische Alterung zu verlangsamen und zumindest nach außen einen ewig jungen Eindruck zu hinterlassen, motivierte unzählige Unternehmen, kosmetische Zusammensetzungen und Wirkstoffe, welche die Hautalterung zu bremsen bzw. zu sogar zu stoppen vermögen, zu erforschen und zu entwickeln. Dabei wurde festgestellt, dass beispielsweise Antioxidantien, wie die Ascorbinsäure (Vitamin C) derartige Eigenschaften aufweisen. Ascorbinsäure ist eine der bekanntesten antioxidativen Substanzen, die aufgrund ausgeprägter antioxidativer Eigenschaften nicht nur in Lebensmitteln, sondern häufig auch in kosmetischen Produkten, insbesondere in den sogenannten "Anti-aging"-Produkten (zur Verlangsamung des natürlichen Alterungsprozesses der Haut), eingesetzt wird. Ascorbinsäure ist an der Hydroxylierung von Prolin im Bereich der Proteinstränge des Kollagenmoleküls beteiligt und führt zur Stimulation des Bindegewebsstoffwechsels mit erhöhter mRNA-Expression der Kollagen synthetisierenden Enzyme und somit auch zur Regeneration der Haut und folglich zur Reduktion der Faltenbildung.

Die Hautpflege, insbesondere die kosmetische Hautglättung, erfolgt in der Regel mit Mitteln, wie Cremen, Gels, Tinkturen und dgl., die Stoffe enthalten, deren komplexe Wirkung auf den Bindegewebsstoffwechsel erforscht wurden und deren positive Wirkungsweise hinlänglich bekannt ist (siehe z.B. Reuther T und Kerscher M., Hautarzt (2004) 55:630-636).

Zur Identifizierung solcher Wirkstoffe werden künstlich gezüchtete Ganzhautmodelle verwendet, mit denen sowohl Wirkstoffe als auch Formulierungen schneller und effektiver als mit herkömmlichen Methoden auf ihre positiven Auswirkungen auf die Haut untersucht werden können.

Mit künstlich gezüchteten In-vitro-Ganzhautmodellen lassen sich beispielsweise die natürlichen Alterungsvorgänge in der Haut experimentell nachstellen und die Einflüsse von Wirkstoffen und Fertigprodukten auf diese Vorgänge wirklichkeitsnah untersuchen. Das verwendete dreidimensionale Hautmodell entsteht innerhalb von fünf Wochen durch sukzessives Züchten einer Dermis aus Fibroblasten und einer Epidermis mit Stratum corneum aus Keratinozyten. Aus diesem differenzierten Aufbau und der Ähnlichkeit zur menschlichen Haut ergibt sich gegenüber konventionellen Fibroblasten-Kulturen oder reinen Epidermis-Modellen eine Reihe von Vorteilen. So besteht zum Beispiel die Möglichkeit der wiederholten Applikation von Wirkstoffen über mehrere Wochen. Außerdem können unter einheitlichen Bedingungen in allen Hautabschnitten biochemische Parameter gemessen werden, die für die Hautalterung von Bedeutung sind (siehe z.B. zur Muhlen A, et al., Skin Pharmacol Physiol. (2004) 17:167-175; Monteiro-Riviere NA, et al., Microsc Res Tech. (1997) 37:172-179).

Das sichtbare Auftreten von Falten, Altersflecken und Schlaffheit findet sein histologisches Korrelat in einer unregelmäßigen Strukturierung der Dermis, einer Atrophie der Epidermis und einem Abflachen der Krümmung an der Grenzfläche zwischen Dermis und Epidermis. Auch der Stoffwechsel der extrazellulären Matrix sowie die Basalmembran weisen mit zunehmendem Alter Veränderungen auf. Aus diesem Grund sind die Regeneration des Bindegewebes effizient fördernde kosmetische Produkte (insbesondere "Anti-aging"-Produkte), die ein möglichst breites Wirkspektrum auf alle relevanten Hautschichten aufweisen, von enormem Interesse.

Ein für den Hautalterungsprozess besonders bedeutsamer Parameter ist der Kollagengehalt in der Dermis. Dieser nimmt pro Lebensjahr um etwa ein Prozent ab, bei gleichzeitigem Anstieg des Anteils an Kollagenasen. Die Folge ist eine Abnahme der Zugfestigkeit der Haut sowie die Bildung von Falten. Ein wesentliches Ziel von Anti-aging-Produkten ist daher die Stimulation der Kollagensynthese. Beispielsweise führte die Verabreichung von Ascorbinsäure (nach viermal täglicher Applikation einer 0,5-prozentigen Gelformulierung) im Hautmodell zu einer Steigerung der Kollagensyntheserate von 40% innerhalb von zehn Tagen. Stabilisiertes Retinol, das als 0,024-%ige Creme unter den gleichen Bedingungen getestet wurde, steigerte die Kollagensyntheserate um 59%.

Retinoide (Derivate des Retinols) sind jedoch - im Gegensatz zu Vitamin C - nicht direkt an der Kollagenbiosynthese beteiligt, sondern wirken als Botenstoffe, die in den Fibroblasten die Kollagensynthese anregen.

Die Rolle von Ascorbinsäure in der kosmetischen Behandlung von Haut wurde vielfach untersucht. So zeigten Meisner und Schnitsky die wesentliche Rolle der L-Ascorbinsäure zur Aufrechterhaltung von jugendlicher Glätte und Elastizität der Haut. Ferner identifizierten die Autoren dieser Studie zwei wichtige chemische "Helfer", nämlich Tyrosin und Zinksulfat, die Hilfestellung dabei leisten, Vitamin C durch die Haut zu transportieren (US 4,938,969; kommerziell als Cellex-C vermarktet).

Die kombinierte Anwendung von Retinol, oder Vitamin A, das in der Haut auf natürliche Weise vorkommt, ist heute in vielen kosmetischen Produkten, insbesondere in Anti-aging-Formulierungen, weit verbreitet. Retinol spielt dabei eine bedeutende Rolle bei der Aufrechterhaltung der Hautglätte und in der Reduktion von feinen Linien, indem es die natürlichen biologischen Prozesse der Zellerneuerung stimuliert. Allerdings nehmen die im Körper produzierten Vitamin-A-Konzentrationen stets ab, da die Haut altert und wiederholt schädlichen UV-Strahlen ausgesetzt ist. Alternde Haut erneuert sich selbst langsamer und die Epidermis wird allmählich dünner.

DE 1069 A betrifft eine Verbandvorrichtung für Kataplasmen, die einen mit einem Filz umgebenen wasserdichten Kautschukbehälter umfasst. Mit dieser Verbandvorrichtung können Wirkstoffe, die auf der Außenseite des Behälters aufgebracht werden, auf die Hautoberfläche bei gleichzeitiger Erwärmung aufgebracht werden.

In der DE 100 02 590 A1 wird eine Gesichtsmaske beschrieben, die geeignet ist, eine Thermobehandlung durchzuführen. Gemäß dieser Schrift liegt die Gesichtsmaske flächig auf dem Gesicht auf, welches zuvor mit einer Creme behandelt wurde, wobei die Gesichtsmaske bei der dem Gesicht zugewandten Seite eine Folie aufweist, welche aus einem Kunststoffmaterial, wie beispielsweise Polyamid, besteht.

Es hat sich in vielen Fällen als schwierig herausgestellt, die in einem kosmetischen Produkt formulierten Wirkstoffe durch die Hautbarriere zu transportieren. Dieser Transport wird weiters durch die Tatsache erschwert, dass eine Vielzahl von topisch verwendeten Kosmetika Stoffe enthalten, die das Eindringen der Wirkstoffe in tiefere Hautschichten, vorzugsweise durch die Poren, verhindern oder derart stark beeinträchtigen, dass eine effiziente kosmetische Behandlung nicht mehr möglich ist, da die Wirkstoffe nicht in den vorgesehenen Wirkungsbereich vordringen können. Vor allem die Verwendung von fetthaltigen Cremen führt dazu, dass die Poren der Haut verstopft werden und das Eindringen von Wirkstoffen, insbesondere von hydrophilen Wirkstoffen, wie Vitamin C, größtenteils unterbunden wird.

Es ist somit Aufgabe der vorliegenden Erfindung, ein neues kosmetisches Verfahren zur Verfügung zu stellen, bei dem kosmetische Wirkstoffe, insbesondere Vitamine, effizienter durch die Haut transportiert werden können und somit eine noch bessere Wirksamkeit erzielt werden kann. Insbesondere sollte sich das Verfahren zur kosmetischen Hautglättung eignen.

Diese Aufgabe wird gelöst durch ein kosmetisches Verfahren zur Hautbehandlung, insbesondere zur Hautglättung und Hautreinigung, umfassend die Schritte:
a) Aufbringen einer flüssigen kosmetischen Zusammensetzung, umfassend ein flüssiges Lösungsmittel ausgewählt aus der Gruppe bestehend aus Wasser, Ethanol und Kombinationen davon und zumindest ein Antioxidans und/oder zumindest ein Vitamin, auf die Haut,
b) Aufbringen einer Creme in Form einer Wasser-in-Öl-Emulsion, eines Gels oder einer Lotion auf die Haut,
c) Abdecken der Haut mit einer Wasser- und Wasserdampf-undurchlässigen kosmetischen Maske, wobei die Haut nach Schritt c) und gegebenenfalls vor Schritt a) wärmebehandelt und die Wasser- und Wasserdampf-undurchlässige kosmetische Maske an der Haut anliegend aufgebracht wird.

Es hat sich in überraschender Weise herausgestellt, dass flüssige kosmetische Zusammensetzungen, welche beispielsweise Vitamine und Antioxidantien umfassen können, durch Wärmebehandlung, die gegebenfalls zu einer Überführung der Zusammensetzung in den dampfförmigen Zustand führt, eine verbesserte Tiefenwirkung auf der Haut bewirken können, sofern die Haut zumindest nach Schritt b) wärmebehandelt wird. Durch die Wärmebehandlung der Haut werden deren Poren geöffnet, wodurch die Haut in der Lage ist, die in der kosmetischen Zusammensetzung befindlichen kosmetischen Wirkstoffe besser und effizienter aufzunehmen. Durch das Aufbringen einer Wasser- und Wasserdampf-undurchlässigen Beschichtung wird verhindert, dass im Zuge der Wärmebehandlung der Haut einerseits die aufgetragene kosmetische Zusammensetzung samt Inhaltsstoffen wegdampft, andererseits die Inhaltsstoffe der Zusammensetzung wieder aus der Haut bzw. den Poren ausgeschwitzt bzw. in die Beschichtung aufgenommen und somit der Haut entzogen werden. Daher ist es von Vorteil, wenn die in der Zusammensetzung befindlichen Stoffe sich nicht in der Beschichtung lösen.

Erfindungsgemäß kann die Haut des gesamten Körpers oder aber auch nur gewisse Hautpartien, wie z.B. das Gesicht oder die Bauchregion, behandelt werden. Jene Hautpartien, die nicht behandelt werden sollen, können beispielsweise durch geeignete Abdeckungen (z.B. Tuch, Folie) versehen werden. Das erfindungsgemäße Verfahren kann nicht nur zur Reduktion von Falten, sondern auch zur Reduktion von Schwangerschaftsstreifen und anderen Hautunebenheiten verwendet werden. Ferner ergibt sich ein Hautreinigender und -regenerierender Effekt.

Aufgrund der Tatsache, dass die Poren der Haut durch die Erwärmung geöffnet werden können, ist es nötig, dass die Haut vor, während und/oder nach dem Inkontaktbringen (z.B. durch Besprühen, Aufenthalt in einer Dampf/Aerosol-haltigen Umgebung) wärmebehandelt wird. Es hat sich erfindungsgemäß gezeigt, dass die Wärmebehandlung der Haut deren Poren öffnet und dadurch die Tiefenwirkung der sich auf der Haut befindlichen Wirkstoffe, insbesondere Vitamine und Antioxidantien, fördert. Vorzugsweise wird die Haut einer Umgebungstemperatur von 25°-60°C, vorzugsweise von 30°-50°C, insbesondere von 35°-40°C, für 2 min bis 2 h, vorzugsweise für 5 min bis 60 min, insbesondere für 10 min bis 40 min, ausgesetzt bzw. vorbehandelt. Die Haut selbst wird für mindestens 2 min, vorzugsweise mindestens 5 min, noch mehr bevorzugt mindestens 10 min, am meisten bevorzugt mindestens 20 min, insbesondere mindestens 30 min der kosmetischen Zusammensetzung ausgesetzt. Die Wärmebehandlung ist jedenfalls so durchzuführen, dass die Haut im Zuge des Verfahrens selbst eine Temperatur von ca. 37°C bis ca. 42°C ausgesetzt wird. Höhere Temperaturen, die direkt auch der Oberfläche der Haut einwirken, können zur Schädigung der Haut selbst führen.

Als Wasser- und Wasserdampf-undurchlässige Beschichtung kann jegliche Art von Beschichtung verwendet werden, sofern diese hautverträglich ist und diese derart auf die Haut aufgebracht werden kann, dass sie an der Haut anliegt und somit konturgleich aufbringbar ist. Als besonders vorteilhaft haben sich dabei kosmetische Marken erwiesen, die insbesondere auf Kalziumsulfat beruhen. Das Anliegen der Beschichtung an der Haut, d.h. das konturgleiche Anliegen an der Haut, gewährt, dass zwischen der Beschichtung und der Haut, worin sich die flüssige Zusammensetzung und die Creme befindet, keine bzw. im Wesentlichen keine Hohlräume ausgebildet werden können. Das Vorhandensein von Hohlräumen, in denen weder Creme noch die flüssige Zusammensetzung vorhanden ist, führt dazu, dass es in diesen Regionen zu Schweißbildung kommt. Eine erhöhte Schweißbildung ist im erfindungsgemäßen Verfahren nachteilig, da dadurch die aufzutragenden Wirkstoffe wieder aus den Poren herausgeschwitzt werden. Zu einer Schweißbildung würde es beispielsweise kommen, wenn Folien verwendet werden, die bekannterweise nicht anliegend auf die Haut aufgebracht werden können.

Es hat sich erfindungsgemäß herausgestellt, dass es von Vorteil ist, eine Creme, die vorzugsweise eine Wasser-in-Öl-Emulsion ist, zu verwenden, da Cremen aufgrund des hohen Öl- bzw. Fettanteils eine reduzierte Wasserdurchlässigkeit aufweisen und dadurch zusätzlich als Wasserdampfbarriere dienen können.

Erfindungsgemäß ist es auch möglich, in der Creme mindestens ein Vitamin oder sonstigen kosmetischen Wirkstoff vorzusehen. Hierbei eignen sich insbesondere Wirkstoffe, die lipophile Eigenschaften aufweisen, wie z.B. Retinol. Daher ist das mindestens eine Vitamin vorzugsweise Retinol.

Die Wärmebehandlung wird vorzugsweise mittels Heißluft oder Dampf durchgeführt.

Es hat sich als vorteilhaft erwiesen, dass die beim Verdampfen einer flüssigen kosmetischen Zusammensetzung entstehende Wärme gleichzeitig zum Öffnen der Poren eingesetzt werden kann. Ferner kann auch eine Infrarotstrahlung eingesetzt werden, die zum einen den Vorteil aufweist, dass dadurch lokale und räumlich begrenzte Teile der Haut (z.B. Infrarotlampe) aber auch große Hautabschnitte (z.B. in einer Infrarotkammer) erwärmt werden können. Eine indirekte Wärmebehandlung der Haut über die Beschichtung mittels Dampf ermöglicht des Weiteren, dass das erfindungsgemäße Verfahren z.B. in einer Sauna, insbesondere in einer Dampfsauna oder in einem Dampfbad, durchgeführt werden kann. Verfahren zur Wärmebehandlung von Haut und Körperpartien mittels Dampf und Infrarotstrahlung sind in der Fachwelt hinreichend bekannt.

Nach der Wärmebehandlung, insbesondere vor Entfernung der Maske, wird die Haut vorzugsweise gekühlt. Die Kühlung kann dabei mittels Kaltluft, Kühlgel oder mit Eis erfolgen. Die Kühlung hat den Vorteil, dass die Poren verschlossen werden und die Wirkstoffe in der Haut verbleiben.

Gemäß einer bevorzugten Ausführungsform der vorliegenden Erfindung umfasst die flüssige kosmetische Zusammensetzung zumindest ein Hormon, insbesondere ein Phytohormon aus einem Pflanzenextrakt, ein Antioxidans und/oder ein Vitamin.

Erfindungsgemäß kann die kosmetische Zusammensetzung ein, zwei, drei, vier, fünf, sechs, sieben, acht oder mehr Vitamine bzw. Vitaminderivate, Hormone und/oder Antioxidantien umfassen.

Die Konzentration der kosmetischen Wirkstoffe in der kosmetischen Zusammensetzung richtet sich nach dem verwendeten Wirkstoff. Erfindungsgemäß werden die Konzentrationsbereiche dabei so gewählt, dass diese herkömmlichen und bekannten kosmetischen Zusammensetzungen entsprechen. So wird z.B. Ascorbinsäure vorzugsweise in einer Konzentration von 0,1 bis 20%, noch mehr bevorzugt in einer Konzentration von 0,5 bis 15%, insbesondere in einer Konzentration von 1 bis 10%, eingesetzt. Retinol wird vorzugsweise in einer Konzentration von 0,01 bis 5%, noch mehr bevorzugt in einer Konzentration von 0,1 bis 1%, eingesetzt. Die kosmetische Zusammensetzung umfasst vorzugsweise 0,1 bis 15%, insbesondere 0,5 bis 10%, α-Tocopherol. Ferner kann die kosmetische Zusammensetzung Panthenol in einer Menge von 0,1 bis 10%, vorzugsweise von 0,5 bis 5%, umfassen. Im Allgemeinen kann die kosmetische Zusammensetzung kosmetische Wirkstoffe in einer Konzentration von 0,01 bis 20% umfassen. Die Wirkstoffe in der kosmetischen Zusammensetzung können auch in Liposomen eingebracht werden.

Gemäß einer weiteren bevorzugten Ausführungsform der vorliegenden Erfindung ist das zumindest eine Vitamin ausgewählt aus der Gruppe bestehend aus Ascorbinsäure, Vitamin B₁, Vitamin B₂, Niacin, Pantothensäure, Vitamin B₆, Biotin, Vitamin B₉, Vitamin B₁₂, α-Tocopherol und Kombinationen davon.

Wasserlösliche Vitamine, wie beispielsweise Ascorbinsäure, aber auch fettlösliche Vitamine, können in tiefere Schichten der Haut kaum vordringen, da die Haut eine natürliche vorwiegend lipophile Barrierefunktion aufweist. Daher eignet sich das erfindungsgemäße Verfahren, welches ein Öffnen der Hautporen bewirkt, den Transport von Vitaminen in tiefere Hautschichten zu ermöglichen bzw. zu erleichtern. Bei herkömmlichen Methoden geht die Verbesserung des Penetrationsverhaltens auf Kosten einer Schädigung der Hautbarriere. Da ein derartiger Ansatz als nicht unproblematisch angesehen werden kann, ist es ratsam das Penetrationsvermögen durch einen möglichst naturnahen, thermischen Prozess zu verbessern. Durch einen derartigen thermischen Prozess werden wie eingangs erwähnt die Poren geöffnet und es wird ermöglicht, dass die Inhaltsstoffe der kosmetischen Zusammensetzung, die sowohl fett- als auch wasserlöslich sein können, in die Haut aufgenommen werden können.

Insbesondere Ascorbinsäure, Retinol, α-Tocopherol, Panthenol und deren kombinierte Verwendung erwiesen sich für das erfindungsgemäße Verfahren als besonders geeignet, da diese Stoffe sowie deren Derivate, wie z.B. Retinoide, einen positiven Einfluss auf die Haut, insbesondere gegen die Faltenbildung, ausüben. Siehe hierzu z.B. Varani J, et al. (J Invest Dermatol. (2000) 114:480-486); Sorg O, et al. (Skin Pharmacol Appl Skin Physiol. (2001) 14:363-372); Glaser DA (Facial Plast Surg Clin North Am. (2004) 12:363-372). Es ist jedoch bevorzugt, fettlösliche Vitamine, wie z.B. Retinol und Retinoide, anstatt in der kosmetischen Zusammensetzung in der auf die Haut aufzutragenden Creme vorzusehen.

Das zumindest eine Antioxidans der erfindungsgemäßen kosmetischen Zusammensetzung ist vorzugsweise Rutin.

Oxidative Prozesse in der Haut, insbesondere Prozesse, die insbesondere durch Umwelteinflüsse (wie z.B. UV-Strahlung, Oxidationsmittel, ionisierende Strahlung) hervorgerufen werden, spielen eine wichtige Rolle bei der Hautalterung, da durch derartige Prozesse Nukleinsäuren, Proteine und Lipide geschädigt werden können. Oxidative Prozesse können überdies so weit führen, dass sich daraus sogar Krankheitszustände, wie z.B. Krebs, ausbilden können. Daher ist es von Vorteil, Antioxidantien, wie z.B. Ascorbinsäure (Vitamin C) und Rutin, auf die Haut mittels des erfindungsgemäßen Verfahrens aufzutragen. Dadurch können oxidative Prozesse in der Haut verhindert bzw. wesentlich reduziert werden.

Gemäß einer bevorzugten Ausführungsform der vorliegenden Erfindung ist der mindestens eine Pflanzenextrakt ausgewählt aus der Gruppe bestehend aus Kigella Africana-Extrakt, Humulus Lupulus-Extrakt, Salvia Officinalis-Extrakt, Alchemilla Vulgaris-Extrakt und Trifolium Pratense-Extrakt.

Phytohormone sind hormonähnliche Substanzen, die in allen höheren Pflanzen hergestellt werden. Die Haut besteht aus drei Schichten, Epidermis, Dermis und Subkutis. Alle sind von Hormonen abhängig, deren Produktion mit dem Älterwerden zurückgeht. Östrogene sorgen beispielsweise für gute Durchblutung und Durchfeuchtung und sind wichtig für die Spannkraft der Haut. Das Gelbkörperhormon Progesteron hingegen regelt die Stabilität der kollagenen Fasern, deren Zustand darüber entscheidet, ob die Haut, insbesondere Gesichtshaut, glatt und prall oder schlaff und faltig ist. Androgene wirken in den Talgdrüsen, Haarfollikeln und im Fettgewebe. Die endogene Hautalterung wird also wesentlich von den Hormonen bzw. deren Fehlen insbesondere nach der Menopause gesteuert, denn in den Wechseljahren beispielsweise sinkt die körpereigene Östrogenproduktion stark ab. Das hat eine Abnahme der Gewebedichte der Haut zur Folge. Um dieser Entwicklung entgegen zu treten, werden vorzugsweise Phythormone in Form von Pflanzenextrakten der kosmetischen Zusammensetzung der vorliegenden Erfindung zugesetzt.

Im erfindungsgemäßen Verfahren werden vorzugsweise nur flüssige kosmetische Zusammensetzungen verwendet, die sich auch in einfacher Weise in einen dampfförmigen Zustand überführen lassen. Derartige Zusammensetzungen bestehen zumeist aus einer in die Dampfform überführbare Matrix, wie z.B. Wasser und/oder Ethanol, und den darin befindlichen Vitaminen und sonstigen kosmetischen Wirkstoffen. Somit umfasst gemäß einer weiteren bevorzugten Ausführungsform der vorliegenden Erfindung die flüssige kosmetische Zusammensetzung ein flüssiges Lösungsmittel ausgewählt aus der Gruppe bestehend aus Wasser, Ethanol und Kombinationen davon. Ferner kann die kosmetische Zusammensetzung, aber auch die Creme, ätherische Öle umfassen.

Die kosmetische Zusammensetzung der vorliegenden Erfindung kann auch in Form eines Gels, insbesondere eines Hydrogels, auf die Haut aufgebracht werden. Die Verwendung von Hydrogelen und deren Zusammensetzungen in der Kosmetik sind in der Fachwelt hinreichend bekannt. Erfindungsgemäß kann jegliches kosmetisch geeignete Hydrogel verwendet werden.

Erfindungsgemäß geeignet sind auch kosmetische Zusammensetzungen in Form einer Lotion oder Tinktur.

Das Verfahren der vorliegenden Erfindung zeichnet sich auch dadurch aus, dass die flüssige kosmetische Zusammensetzung in ein Aerosol, vorzugsweise durch einen Flüssigkeitszerstäuber, ausgewählt aus der Gruppe Druckzerstäuber, Druckluftzerstäuber, Fliehkraftzerstäuber, elektrostatischer Zerstäuber und Ultraschallzerstäuber überführt und auf die Haut aufgetragen werden kann.

Verfahren und Vorrichtungen zur Überführung von Flüssigkeiten, wie die erfindungsgemäßen flüssigen kosmetischen Zusammensetzungen in ein Aerosol sind dem Fachmann hinreichend bekannt und im Stand der Technik ausreichend beschrieben. Vorzugsweise wird das Aerosol als Nebel zerstäubt. Aus der US 4,771,608 ist eine Vorrichtung zur Erzeugung von Nebel bekannt, bei der in einem großen erhitzten Behälter Dampf erzeugt wird, der über eine Leitung in einen Behälter mit flüssigem Kältemittel gelangt, über die freiliegende Oberfläche des Kältemittels geleitet wird und sich dabei in einen Nebel umwandelt, der Wassertröpfchen enthält. Die Nebelerzeugung kann dabei durch mehr oder weniger weites Einschieben eines Dampfabgaberohrs in den Kältemittelbehälter in der Weise beeinflusst werden, dass sich der Abstand des Dampfaustritts aus dem Rohr zur Kältemitteloberfläche ändert. Zusätzlich ist auch noch ein Regelventil in der Dampfleitung vorgesehen. In der DE 16 043 05 wird eine Ultraschall-Flüssigkeitsnebel erzeugende Vorrichtung offenbart, die sich ebenfalls zum Einsatz in einem Verfahren gemäß der vorliegenden Erfindung eignet.

In einer bevorzugten Ausführungsform ist die Wasser- und Wasserdampf-undurchlässige Beschichtung eine kosmetische Maske. Diese Maske dient insbesondere dazu, die auf der kosmetischen Zusammensetzung aufgebrachten Beschichtung zu stabilisieren. Im Gegensatz zu Folien weisen Masken den Vorteil auf, dass sie in der Lage sind, sich den Konturen einer Hautoberfläche anzupassen und sich dadurch an die Haut anlegen.

Besonders bevorzugte Masken umfassen zumindest einen Stoff ausgewählt aus der Gruppe bestehend aus Alginat, Kalziumsulfat und Natriumphosphat.

Gemäß einer bevorzugten Ausführungsform der vorliegenden Erfindung wird vor Schritt a) und/oder nach Schritt c) die Haut mit einer Pflegecreme behandelt. Durch die Verwendung von Pflegecremen kann die Haut vor dem Aufbringen der kosmetischen Zusammensetzung auf ihr vorkonditioniert werden.

### BEISPIELE:

Die folgenden Vergleichsbeispiele belegen, dass die Wirksamkeit einer erfindungsgemäß durchgeführten kosmetischen Behandlung zu einer verbesserten kosmetischen Wirksamkeit führen. Dabei wurde in Beispiel 1 eine erfindungsgemäße flüssige kosmetische Zusammensetzung einer auf Vaseline basierenden Creme gegenübergestellt. In Beispiel 2 wurde eine zur erfindungsgemäßen Maske alternative Maske verwendet.

### Beispiel 1:

In diesem Beispiel wurden 2 Testpersonen mit dem erfindungsgemäßen Verfahren behandelt und zwei weitere mit einem Verfahren bei dem anstelle der flüssigen kosmetischen Zusammensetzung ausschließlich eine auf Vaseline basierende Creme auf die Haut aufgebracht wurde.

Die flüssige kosmetische Zusammensetzung auf Basis von Wasser und Alkohol hatte folgende Zusammensetzung (lt. INCI): AQUA (WA-TER), ALCOHOL, TOCOPHERYL ACETATE (VITAMIN E), HAMAMELIS VIRGI-NIANA (WITCH HAZEL) DISTILLATE, ASCORBIC ACID, LIMONENE, PARFUM, RETINYL PALMITATE, wobei die Konzentration von Vitamin E 1%, von Ascorbinsäure 0,4% und von Retinyl-Palmitat 0,04% betrug.

Um einen direkten Vergleich anzustellen enthielt die Vaseline-haltige Creme u.a. ebenfalls 1% Vitamin E, 0,4% Ascorbinsäure und 0,04% Retinyl-Palmitat.

Die flüssige Zusammensetzung und die Vaseline-haltige Creme wurde auf das Gesicht, einschließlich Augenlider, Nase und Lippen, von je zwei Testpersonen aufgebracht. Anschließend wurde das Gesicht bis auf die Nasenöffnungen vollkommen mit einer Gesichtsmaske auf Alginatbasis beschichtet. Nach dem Aushärten der Gesichtsmaske wurde die Gesichtsmaske und infolge dessen die Hautoberfläche mittels Heißluft erwärmt. Die Behandlung mit Heißluft erfolgte über einen Zeitraum von 30 Minuten.

Es wurden pro Testperson insgesamt 4 Behandlungen über einen Zeitraum von 3 Wochen durchgeführt.

*Ergebnisse:*
a) Testpersonen 1 und 2 behandelt mit einer Vaseline-haltigen Creme (Zusammensetzung siehe oben):
   i) vor der ersten Behandlung:
      Der allgemeine Zustand der Haut wirkte müde und durch die Winterluft auch unregelmäßig durchblutet. Besonders auffällig waren die Bereiche um den Mund (Nasolabial-Falte) und die Trockenheitsfalten seitlich der Augen (Krähenfüßchen). Die Zornesfalte zwischen den Augen war ansatzweise ausgeprägt. Bei einer Testperson war im Kinnbereich leichte Akne erkennbar.
   ii) nach der ersten Behandlung:
      Nach der ersten Behandlung wirkte das Hautbild deutlicher durchblutet, besonders bei den Wangenpartien. Der Effekt war jedoch nur von kurzer Dauer, denn jeweils vor den drei weiteren Behandlungen war das Hautbild im Wesentlichen ident wie vor der ersten Behandlung (siehe i)). Es konnte somit mit der Vaseline-haltigen Creme kein nachhaltiger Erfolg erzielt werden. Bei der Testperson mit Akne im Kinnbereich wurde sogar beobachtet, dass diese nach der zweiten und dritten Behandlung verstärkt wurde.
b) Testpersonen 3 und 4 behandelt mit einer flüssigen kosmetischen Zusammensetzung (siehe oben):
   i) vor der ersten Behandlung:
      Der allgemeine Zustand der Haut wirkte auch bei diesen beiden Testpersonen müde und schlaff. In Teilbereichen war die Haut durch die Winterluft unregelmäßig durchblutet. Die Faltenbildung ist jedoch im Bereich der Nasolabial-Falte mit Verlauf zum Kinn, Augenpartie mit Krähenfüßchen und Zornsfalte zwischen den Augenbrauen verstärkt sichtbar. Beide Testpersonen hatten Bereiche im Gesicht (Wangen und Stirn) mit leichter Akne.
   ii) nach der ersten Behandlung:
      Bereits nach der ersten Behandlung wirkte das Hautbild erfrischender und deutlich durchbluteter als zuvor. Die schlaff wirkenden Hautpartien wirkten nach der ersten Behandlung bereits teilweise straffer, wobei sich der Behandlungseffekt mit der Anzahl der Behandlungen immer mehr zeigte. Sogar zwischen den Behandlungen konnte der Behandlungserfolg beibehalten werden. Im Gegensatz zu den Ergebnissen mit der Vaseline-haltigen Creme konnte bei einer Testperson die Akne auf der Stirn und den Wangen signifikant reduziert werden.

### Beispiel 2:

In diesem Beispiel wurden 2 Testpersonen mit dem erfindungsgemäßen Verfahren mit der in Beispiel 1 angeführten flüssigen kosmetischen Zusammensetzung behandelt. Zwei weiteren Testpersonen wurde anstelle der erfindungsgemäßen Gesichtsmaske eine herkömmliche Vliesmaske aufgebracht.

Die weiteren Behandlungsschritte entsprechen jenen aus Beispiel 1.

*Ergebnisse:*
a) Testpersonen 5 und 6 deren Gesicht mit einer herkömmlichen Vliesmaske bedeckt wurde:
   i) vor der ersten Behandlung:
      Die Testpersonen 5 und 6 hatten ein schwaches Bindegewebe, was deutlich sichtbar durch die Faltenbildung im gesamten Gesicht zu sehen ist. Auch war der Tonus der Haut sehr schwach und die Haut wirkte sehr müde.
   ii) nach der ersten Behandlung:
      Eine Reduktion der Falten konnte nur punktuell nach drei Behandlungen beobachtet werden. Dies hängt damit zusammen, dass die verwendete Maske nicht an der Haut anliegend aufgebracht werden konnte und Öffnungen bei den Augen, Mund und Nase vorhanden waren. Die Haut wirkte auch nicht einheitlich frischer und durchbluteter. Die Bereiche um die Öffnungen der Maske, insbesondere um die Augen, waren im Wesentlichen ident mit dem Zustand vor der ersten Behandlung.
b) Testpersonen 7 und 8 deren Gesicht mit der erfindungsgemäßen Maske bedeckt wurde:
   i) vor der ersten Behandlung:
      Der allgemeine Zustand der Haut dieser Testpersonen wirkte eher müde und durch die Winterluft unregelmäßig durchblutet. Auffällig waren auch die Bereiche um den Mund (Nasolabial-Falte) und die Krähenfüßchen seitlich der Augen.
   ii) nach der ersten Behandlung:
      Die vermehrte Durchblutung im Wangen, Nasen und Oberlippenbereich war deutlich und schön sichtbar. Dadurch war die Prallheit der Haut bereits nach der ersten Behandlung als eindeutige Aktivierung erkennbar. Die Augenfalten wurden nach der vierten Behandlung ebenfalls signifikant reduziert. Die vorteilhaften Wirkungen der erfindungsgemäßen Maske waren im Vergleich zu alternativen Gesichtsmasken insbesondere gut im Augenbereich sichtbar. Schön erkennbar war auch der stark verbesserte Verlauf der Nasolabial-Falte mit Verlängerung zum Kinn.

## Patentansprüche

1. Kosmetisches Verfahren zur Hautbehandlung, insbesondere zur Hautglättung und Hautreinigung, umfassend die Schritte:
a) Aufbringen einer flüssigen kosmetischen Zusammensetzung auf die Haut,
b) Aufbringen einer Creme in Form einer Wasser-in-Öl-Emulsion, eines Gels oder einer Lotion auf die Haut,
c) Abdecken der Haut mit einer Wasser- und Wasserdampf-undurchlässigen Beschichtung, wobei die Haut nach Schritt c) und gegebenenfalls vor Schritt a) wärmebehandelt und die Wasser- und Wasserdampf-undurchlässige Beschichtung an der Haut anliegend aufgebracht wird.

2. Verfahren nach Anspruch 1, **dadurch gekennzeichnet, dass** die Wärmebehandlung mittels Heißluft, beheizbarer Maske, Dampf oder Infrarotstrahlung durchgeführt wird.

3. Verfahren nach Anspruch 1 oder 2, **dadurch gekennzeichnet, dass** die flüssige kosmetische Zusammensetzung zumindest ein Hormon, insbesondere ein Phytohormon aus einem Pflanzenextrakt, ein Antioxidans und/oder ein Vitamin umfasst.

4. Verfahren nach Anspruch 3, **dadurch gekennzeichnet, dass** das zumindest eine Vitamin ausgewählt ist aus der Gruppe bestehend aus Ascorbinsäure, Vitamin B₁, Vitamin B₂, Niacin, Pantothensäure, Vitamin B₆, Biotin, Vitamin B₉, Vitamin B₁₂, α-Tocopherol und Kombinationen davon.

5. Verfahren nach Anspruch 3, **dadurch gekennzeichnet, dass** das zumindest eine Antioxidans Rutin ist.

6. Verfahren nach Anspruch 3, **dadurch gekennzeichnet, dass** der zumindest eine Pflanzenextrakt ausgewählt ist aus der Gruppe bestehend aus Kigella Africana-Extrakt, Humulus Lupulus-Extrakt, Salvia Officinalis-Extrakt, Alchemilla Vulgaris-Extrakt und Trifolium Pratense-Extrakt.

7. Verfahren nach einem der Ansprüche 1 bis 6, **dadurch gekennzeichnet, dass** die flüssige kosmetische Zusammensetzung ein flüssiges Lösungsmittel ausgewählt aus der Gruppe bestehend aus Wasser, Alkohol, insbesondere Ethanol, und Kombinationen davon umfasst.

8. Verfahren nach einem der Ansprüche 1 bis 7, **dadurch gekennzeichnet, dass** die flüssige kosmetische Zusammensetzung in Form eines Aerosols, erzeugbar durch einen Flüssigkeitszerstäuber ausgewählt aus der Gruppe Druckzerstäuber, Druckluftzerstäuber, Fliehkraftzerstäuber, elektrostatischer Zerstäuber und Ultraschallzerstäuber, aufgebracht wird.

9. Verfahren nach einem der Ansprüche 1 bis 8, **dadurch gekennzeichnet, dass** die Creme, das Gel oder die Lotion mindestens ein Vitamin umfasst.

10. Verfahren nach Anspruch 9, **dadurch gekennzeichnet, dass** das mindestens eine Vitamin ausgewählt ist aus der Gruppe bestehend aus Retinol und Derivaten davon.

11. Verfahren nach einem der Ansprüche 1 bis 12, **dadurch gekennzeichnet, dass** die Wasser- und Wasserdampf-undurchlässige Beschichtung eine kosmetische Maske ist.

12. Verfahren nach Anspruch 11, **dadurch gekennzeichnet, dass** die kosmetische Maske zumindest einen Stoff ausgewählt aus der Gruppe bestehend aus Alginat, Kalziumsulfat und Natriumphosphat umfasst.

13. Verfahren nach einem der Ansprüche 1 bis 12, **dadurch gekennzeichnet, dass** vor Schritt a) und/oder nach Schritt c) die Haut mit einer Pflegecreme behandelt wird.

14. Verfahren nach einem der Ansprüche 1 bis 13, **dadurch gekennzeichnet, dass** nach der Wärmebehandlung der Haut in Schritt c) die Haut gekühlt wird.
